# EUROPÄISCHE PATENTSCHRIFT

(11) **EP 1 242 493 B1**
(45) Veröffentlichungstag und Bekanntmachung des Hinweises auf die Patenterteilung: **08.10.2003**
(21) Anmeldenummer: 00985164.3
(22) Anmeldetag: 15.12.2000
(51) Int. Cl.: C08G 18/62, A61K 6/087, C08G 18/67

(54) **RADIKALISCH HÄRTBARE URETHANPRÄPOLYMERE UND DEREN VERWENDUNG IN DENTALMASSEN**
RADICALLY CURABLE URETHANE PREPOLYMERS AND THEIR USE IN DENTAL MATERIALS
PREPOLYMERES D'URETHANNE A DURCISSEMENT RADICALAIRE ET LEUR UTILISATION COMME PRODUITS DENTAIRES

(30) Priorität: 17.12.1999 DE 19961342
(43) Veröffentlichungstag der Anmeldung: 25.09.2002
(73) Patentinhaber: ESPE Dental AG, 82229 Seefeld (DE)
(72) Erfinder: HECHT, Reinhold, 86916 Kaufering (DE); LECHNER, Günther, 82237 Wörthsee (DE); LEHMANN, Thomas, 84489 Burghausen (DE); ECKHARDT, Gunther, 06231 Bad Düerrenberg (DE); GANGNUS, Bernd, 82346 Andechs (DE)
(74) Vertreter: Freiherr von Wittgenstein, Arved, Dr.
(86) Internationale Anmeldenummer: EP0012775
(87) Internationale Veröffentlichungsnummer: WO01044338

(56) Entgegenhaltungen:
- EP-A- 0 205 846
- EP-A- 0 257 777
- EP-A- 0 460 478
- EP-A- 0 945 469

## Beschreibung

Die vorliegende Erfindung beschreibt di- oder höherfunktionelle radikalisch härtbare Urethanpräpolymere und deren Einsatz in polymerisierbaren Massen. Die Erfindung beschreibt weiterhin die Verwendung der polymerisierbaren Massen im Dentalbereich, beispielsweise als Füllungsmaterialien, Stumpfaufbaumaterialien, Befestigungszemente, provisorische Kronen- und Brückenmaterialien, zahntechnische Werkstoffe, Modellmaterialien oder zur Herstellung von Inlays, Onlays, Verblendschalen, Kronen und Brücken.

Für solche Anwendungszwecke geeignete Materialien weisen vorzugsweise eine hohe Schlagzähigkeit, hohe Elastizität bei hoher Härte sowie eine geringe Quellneigung aufweisen. Diese Eigenschaften werden im wesentlichen durch die verwendeten Monomere bestimmt.

Im Stand der Technik werden als geeignete Monomere vor allem ethylenisch ungesättigte Verbindungen, wie Acrylsäure- und/oder Methacrylsäureester, beschrieben.

Speziell aus der Gruppe der Urethan(meth)acrylate wird beispielsweise in der Dentalindustrie im wesentlichen 7,7,9-Trimethyl-4,13-dioxo-3,14,-dioxa-5,12-diazahexadecan-1,16-dioxy-dimethacrylat (z.B. Plex 666-1, Röhm) verwendet, welches mit seinem niedrigen Molekulargewicht wenig elastifizierend wirkt und die Schlagzähigkeit nur wenig verbessert.

Um die elastifizierenden Eigenschaften und die Schlagzähigkeit zu verbessern werden beispielsweise in der Lackindustrie bei radikalisch härtbaren Systemen eine Vielzahl von mit (Meth)acrylatgruppen endfunktionalisierten Polyurethanoligomeren verwendet, die Polyester-, Polyether-, Polybutadienund/oder Polycarbonat-Einheiten besitzen. Jedoch weisen diese Systeme eine Reihe von Nachteilen auf:

So neigen die Esterbindungen der Polyester-Urethan(meth)acrylate und der Polycarbonat-Urethan(meth)acrylate bei Kontakt mit Feuchtigkeit zur Hydrolyse, wodurch insbesondere die mechanischen Werte daraus hergestellter Formulierungen herabgesetzt werden.

Urethan(meth)acrylate auf Polyetherbasis zeigen eine deutlich geringere Anfälligkeit gegen Hydrolyse. Allerdings weisen diese Systeme aufgrund ihrer Hydrophilie eine vermehrte Wasseraufnahme und damit eine erhöhte Quellneigung und geringere Festigkeit auf, die mit einer leichten Verfärbbarkeit einhergeht. Solche Systeme sind daher für ästhetisch anspruchsvolle Anwendungen nicht geeignet.

Ein weiterer Nachteil der Polyether-Urethan(meth)acrylate liegt in deren mangelnder Oxidationsbeständigkeit in Folge des oxidativen Polyetherkettenabbaus.

Die Polybutadien-Urethan(meth)acrylate zeigen zwar hervorragende elastische Eigenschaften, doch werden sie durch die vorhandenen Doppelbindungen leicht durch kurzwellige Strahlung, wie sie im Sonnenlicht enthalten ist, verfärbt.

Prinzipiell können auch aus niedermolekularen aliphatischen und/oder aromatischen Diolen Urethan(meth)acrylate hergestellt werden. Diese zeigen jedoch nicht so gute elastische und schlagzähmodifizierende Eigenschaften, wie die obengenannten Urethan(meth)acrylate, die Weichsegmente auf Polyester-, Polyether-, Polybutadien- und/oder Polycarbonateinheiten enthalten.

Es besteht daher ein erheblicher Bedarf an Monomeren, die in härtbaren Massen zu einer Verbesserung der Schlagzähigkeit führen, gute Elastizität bei hoher Härte aufweisen und nur wenig quellen.

Aufgabe der vorliegenden Erfindung ist es, Monomere zur Verfügung zu stellen, aus denen Massen formuliert werden können, die eine verbesserte Schlagzähigkeit, bei gleichzeitig hoher Härte und guter Elastizität aufweisen.

Überraschenderweise wurde gefunden, dass diese Anforderungen mit Urethanpräpolymeren erreicht werden, die erhältlich sind durch Umsetzung von:
(A) 15 bis 85 Gew.-%, bevorzugt 20 bis 80 Gew.-% eines oder mehrerer α,ω-terminierter Poly(meth)acrylatdiole,
(B) 0 bis 30 Gew.-%, bevorzugt 0 bis 20 Gew.-% einer oder mehrerer radikalisch härtbarer, polyhydroxyfunktioneller Verbindungen,
(C) 14 bis 60 Gew.-%, bevorzugt 18 bis 50 Gew.-% eines oder mehrerer Polyisocyanate,
(D) 1 bis 40 Gew.-%, bevorzugt 2 bis 35 Gew.-% einer gegenüber Isocyanatgruppen reaktiven monofunktionellen Verbindung, die zusätzlich über eine oder mehrere radikalisch härtbare Gruppierungen verfügt.

Überraschenderweise zeigen Massen, die mit den erfindungsgemäßen Monomeren formuliert wurden, eine extrem geringe Quellung.

Ferner sind die Monomere toxikologisch unbedenklich und eignen sich daher besonders für den Einsatz im Dentalbereich.

Bei den α,ω-terminierten Poly(meth)acrylatdiolen der Komponente (A) handelt es sich um Verbindungen, die zwei endständige Hydroxylgruppen aufweisen. Geeignete Ausgangsverbindungen zum Aufbau dieser α,ω-terminierten Poly(meth)acrylatdiole und das Herstellverfahren für diese Verbindungen werden in der EP-B1-0 622 378 bzw. EP-B1-0 205 846 ausführlich beschrieben. Unter der Bezeichnung "Tego-Diol" sind sie von der Firma Tego kommerziell erhältlich.

Bei der Komponente (B) handelt es sich um radikalisch härtbare Verbindungen, beispielsweise auf (Meth)acrylatbasis, die gemäß DIN 53 240 OH-Zahlen bevorzugt von 40 bis 700 mg KOH/g und besonders bevorzugt von 80 bis 500 mg KOH/g aufweisen.

Geeignete Vertreter sind beispielsweise polyhydroxygruppenhaltige Polyester(meth)acrylatpräpolymere, wie sie in der US-A-4 206 205, DE-OS-40 40 290, DE-OS-33 16 592, DE-OS-37 04 098 und in "UV & EB Curing Formulations for Printing Inks Coatings and Paints", ed. R. Holman and P. Oldring, published by SITA Technology, London (England) 1988, S. 36f. beschrieben werden.

Alternativ können auch polyhydroxygruppenhaltige Polyepoxy(meth)acrylatpräpolymere, die durch Umsetzung von Polyepoxiden mit (Meth)acrylsäure zugänglich sind, und/oder polyhydroxygruppenhaltige Polyurethan(meth)acrylatpräpolymere eingesetzt werden.

Besonders bevorzugte Vertreter sind polyhydroxygruppenhaltige Polyepoxy(meth)acrylatpräpolymere, wie 2,2-Bis-[p-(2'-hydroxy-3'methacryloyloxypropoxy)-phenyl]-propan (Bis-GMA) oder 2,2-Bis-[p-(2'-hydroxy-3'-acryloyloxypropoxy)-phenyl]-propan (Bis-GA) und polyhydroxygruppenhaltige (Meth)acrylatester, wie Glycerinmono(meth)acrylat, Trimethylolpropanmono(meth)acrylat oder Pentaerythritdi(meth)acrylat.

Geeignete Polyisocyanate der Komponente (C) sind aliphatischer, cycloaliphatischer und/oder aromatischer Natur und weisen mindestens zwei freie Isocyanatgruppen auf. Vorzugsweise werden Diisocyanate X(NCO)₂ eingesetzt, wobei X einen aliphatischen Kohlenwasserstoffrest mit 2 bis 12 C-Atomen, einen cycloaliphatischen Kohlenwasserstoffrest mit 5 bis 18 C-Atomen, einen aromatischen Kohlenwasserstoffrest mit 6 bis 16 C-Atomen und/oder einen araliphatischen Kohlenwasserstoffrest mit 7 bis 15 C-Atomen bedeutet.

Beispiele für derartige Diisocyanate sind: 1,4-Tetramethylendiisocyanat, 1,6-Hexamethylendiisocyanat (HDI), 2,4,4-Trimethyl-hexamethylendiisocyanat, Isophorondiisocyanat, 4,4'-Dicyclohexylmethandiisocyanat, meta- und para-Tetramethylxyloldiisocyanat, 1,4-Phenylendiisocyanat, 2,6- und 2,4-Toluoldiisocyanat, 1,5-Naphthylendiisocyanat, 2,4' und 4,4'-Diphenylmethandiisocyanat.

Es ist selbstverständlich auch möglich, die aus der Polyurethanchemie bekannten höherfunktionellen Polyisocyanate oder auch modifizierte, beispielsweise Carbodiimidgruppen-, Allophanatgruppen-, Isocyanuratgruppen und/oder Biuretgruppen aufweisende Polyisocyanate einzusetzen bzw. anteilig mitzuverwenden. Besonders bevorzugte lsocyanate sind Isophorondiisocyanat und 2,4,4-Trimethyl-hexamethylendiisocyanat.

Verbindungen der Komponente (D) weisen eine gegenüber Isocyanaten reaktive funktionelle Gruppe auf, beispielsweise eine Hydroxygruppe oder Aminogruppe, und verfügen darüber hinaus über eine bzw. mehrere radikalisch härtbare Gruppierungen. Die radikalisch härtbaren Gruppierungen sind bevorzugt auf (Meth)acrylatbasis. Die Vertreter dieser Komponente (D) können auch als Abmischung eingesetzt werden.

Geeignete Vertreter sind beispielsweise 2-Hydroxyethyl(meth)acrylat, 2-Hydroxypropyl(meth)acrylat, Glycerindi(meth)acrylat und/oder Trimethylolpropandi(meth)acrylat. Besonders bevorzugt sind 2-Hydroxyethylmethacrylat (HEMA) und/oder 2-Hydroxyethylacrylat (HEA).

Zur Herstellung der erfindungsgemäßen Urethanpräpolymere werden beispielsweise die Komponenten (A) bis (C) in einem Reaktor vorgelegt oder einzeln zudosiert und unter wasserfreien Bedingungen, beispielsweise in einem Temperaturbereich von -20°C bis 160°C, bevorzugt in einem Temperaturbereich von 0°C bis 130°C und besonders bevorzugt einem Temperaturbereich von 20°C bis 100°C zu einem NCO-haltigen Präpolymeren umgesetzt.

Das Äquivalentverhältnis von Isocyanatgruppen zu gegenüber Isocyanatgruppen reaktiven Verbindungen beträgt 1,1 : 1 bis 8:1, bevorzugt 1,5 : 1 bis 4 : 1.

Die Isocyanatpolyadditionsreaktion kann in Gegenwart von aus der Polyurethanchemie bekannten Katalysatoren, beispielsweise Organo-Zinn-Verbindungen, wie Dibutylzinndilaurat oder Aminkatalysatoren, wie Diazabicyclo[2.2.2]octan, erfolgen. Weiterhin kann die Synthese sowohl in der Schmelze als auch in einem geeigneten Lösungsmittel, welches vor oder während der Präpolymerherstellung zugesetzt werden kann, erfolgen. Geeignete Lösungsmittel sind beispielsweise Aceton, 2-Butanon, Tetrahydrofuran, Dioxan, Dimethylformamid, N-Methyl-2-pyrrolidon (NMP), Ethylacetat, Alkylether von Ethylen- und Propylenglykol und aromatische Kohlenwasserstoffe. Besonders bevorzugt ist der Einsatz von Ethylacetat als Lösungsmittel.

Zu den NCO-haltigen Präpolymeren wird unter Rühren die Komponente (D) komplett oder portionsweise zudosiert und beispielsweise in einem Temperaturbereich von -20°C bis 160°C, bevorzugt in einem Temperaturbereich von 0°C bis 130°C und besonders bevorzugt einem Temperaturbereich von 20°C bis 100°C zur Reaktion gebracht. Die Menge an zu verwendender Komponente (D) hängt von den noch vorhandenen, nicht umgesetzten Isocyanatgruppen des Präpolymeren ab. Die Bestimmung des Isocyanatgehalts des Präpolymeren erfolgt beispielsweise gemäß DIN 53 185.

Beispielsweise kann das aus GPC-Messungen gegen Polystyrolstandards erhaltene Gewichtsmittel des Molekulargewichts (M_{w}) zwischen 400 und 200.000 g/mol, bevorzugt zwischen 500 und 100.000 g/mol und besonders bevorzugt zwischen 600 und 50.000 g/mol liegen. Natürlich können die Molekulargewichte auch außerhalb dieser Bereiche liegen, soweit es die Anwendung der erfindungsgemäßen Polyurethanpräpolymere zulässt.

Die erfindungsgemäßen Urethanpräpolymere eignen sich beispielsweise zur Herstellung von härtbaren Massen, wie Dentalmassen oder zum Beschichten, Vergießen und Verkleben von Substraten.

Härtbare Formulierungen, beispielsweise für die Verwendung im Dentalbereich, enthalten bevorzugt folgende Komponenten:
(K1) 1 bis 70 Gew.-%, insbesondere 2 bis 50 Gew.-% erfindungsgemäßes Urethanpräpolymer,
(K2) 8,9 bis 70 Gew.-%, insbesondere 10 bis 60 Gew.-% eines oder mehrerer radikalisch polymerisierbarer Monomerer,
(K3) 10 bis 90 Gew.-%, insbesondere 10 bis 87,9 Gew.-% Füllstoffe,
(K4) 0,1 bis 5 Gew.-%, insbesondere 0,1 bis 3 Gew.-% Initiatoren und gegebenenfalls Aktivatoren,
(K5) 0 bis 30 Gew.-%, insbesondere 0 bis 20 Gew.-% Additive, gegebenenfalls Pigmente Thixotropiehilfsmittel, Weichmacher.

Die mit den erfindungsgemäßen Urethanpräpolymeren formulierten Massen zeichnen sich durch besonders gute Schlagzähigkeit, gute Elastizität bei hoher Härte und überraschend geringe Quellneigung aus.

Als Komponente (K2) werden mono-, di-, oder höherfunktionelle ethylenisch ungesättigte Verbindungen, bevorzugt auf Acrylat- und/oder Methacrylatbasis eingesetzt. Diese können sowohl monomere als auch höhermolekulare oligomere oder polymere Acrylate beinhalten. Ferner können sie in alleiniger Form oder in Abmischung in den Formulierungen eingesetzt werden.

Geeignete Monomere sind beispielsweise die Acrylsäure- und Methacrylsäureester mono-, di- oder höherfunktioneller Alkohole. Exemplarisch seien genannt: Methyl(meth)acrylat, iso-Butyl(meth)acrylat, Ethylenglykoldi(meth)acrylat, Triethylenglykoldi(meth)acrylat (TEGDMA), Hexandioldi(meth)acrylat, Dodecandioldi(meth)acrylat und Trimethylolpropantri(meth)acrylat.

Vorteilhaft einsetzbar ist weiterhin Bisphenol-A-di(meth)acrylat sowie die davon abgeleiteten ethoxy- bzw, propoxylierten Di(meth)acrylate. Auch die in der US-A-3 066 112 beschriebenen Monomere auf Basis von Bisphenol-A und Glycidyl(meth)acrylat oder deren durch Addition von Isocyanaten entstandenen Derivate sind geeignet.

Besonders geeignet sind auch die in der DE-C-28 16 823 genannten Diacryl- und Dimethacrylsäureester des Bis[hydroxymethyl)-tricyclo[5.2.1.0^{2,6}]-decans und die Diacryl- und Dimethacrylsäureester der mit 1 bis 3 Ethylenoxid- und/oder Propylenoxideinheiten verlängerten Verbindungen des Bis(hydroxymethyl)-tricyclo[5.2.1.0^{2.6}]-decans.

Auch Urethan(meth)acrylate, wie 7,7,9-Trimethyl-4,13-dioxo-5,12-diazahexadecan-1,16-dioxy-dimethacrylat (UDMA), können Bestandteil der Komponente (K2) sein.

Füllstoffe gemäß Komponente (K3) können anorganische Füllstoffe, beispielsweise Quarz, gemahlene Gläser, nicht wasserlösliche Fluoride wie CaF₂, Kieselgele sowie Kieselsäure, insbesondere pyrogene Kieselsäure und deren Granulate sein. Auch Christobalit, Calciumsilikat, Zirkoniumsilikat, Zeolithe, einschließlich der Molekularsiebe, Metalloxidpulver, wie Aluminium- oder Zinkoxide bzw. deren Mischoxide, Bariumsulfat, Yttriumfluorid, Calciumcarbonat sind als Füllstoffe einsetzbar.

Auch fluoridauslösende Füllstoffe, beispielsweise komplexe anorganische Fluoride der allgemeinen Formel AₙMFₘ wie in der DE-A-44 45 266 beschrieben, können eingesetzt bzw. zugesetzt werden. Dabei bedeutet A ein ein- oder mehrwertiges Kation, M ein Metall der III, IV, V Haupt- oder Nebengruppe, n eine ganze Zahl von 1 bis 3 und m eine ganze Zahl von 4 bis 6.

Bestandteil der Komponente (K3) können auch organische Füllstoffe sein. Exemplarisch genannt seien übliche perlförmige Polymere und Copolymere auf Basis von Methylmethacrylat, die beispielsweise bei der Fa. Röhm unter der Bezeichnung "Plexidon" oder "Plex" erhältlich sind.

Zum besseren Einbau in die Polymermatrix kann es von Vorteil sein, die genannten Füllstoffe sowie gegebenenfalls röntgenopake Zusatzstoffe zu hydrophobieren. Die Menge des eingesetzten Silans beträgt üblicherweise 0,5 bis 10 Gew.-%, bezogen auf anorganische Füllstoffe, bevorzugt 1 bis 6 Gew.-%, ganz besonders bevorzugt 2 bis 5 Gew.-%, bezogen auf anorganische Füllstoffe. Übliche Hydrophobierungsmittel sind Silane, beispielsweise Trimethoxymethacryloxypropylsilan. Die maximale mittlere Korngröße der anorganischen Füllstoffe beträgt vorzugsweise 15 µm, insbesondere 8 µm. Ganz besonders bevorzugt werden Füllstoffe mit einer mittleren Korngröße von < 3 µm eingesetzt.

Unter Initiatoren gemäß Komponente (K4) sind Initiatorsysteme, die die radikalische Polymerisation der Monomeren bewirken, beispielsweise Photoinitiatoren und/oder sogenannte Redoxinitiatorsysteme und/oder thermische Initiatoren zu verstehen.

Als Photoinitiatoren eignen sich beispielsweise α-Diketone, wie Campherchinon, in Verbindung mit sekundären und tertiären Aminen, oder Mono- und Bisacylphosphinoxide, wie 2,4,6-Trimethylbenzoyldiphenyl-phosphinoxid und Bis-(2,6-dichlorbenzoyl)-4-n-propylphenyl-phosphinoxid. Es eignen sich aber auch andere Verbindungen dieses Typs, wie sie in den europäischen Patentveröffentlichungsschriften EP-A-0 073 413, EP-A-0 007 508, EP-A-0 047 902, EP-A-0 057 474 und EP-A-0 184 095 beschrieben sind.

Als Redoxinitiatorsysteme eignen sich organische Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoylperoxid und p-Methylbenzoylperoxid in Betracht.

Als Aktivatoren eignen sich beispielsweise tertiäre aromatische Amine, wie die aus der US-A-3 541 068 bekannten N,N-Bis-(hydroxyalkyl)-3,5-xylidine sowie die aus der DE-A-26 58 530 bekannten N,N-Bis-(hydroxyalkyl)-3,5-di-t-butylaniline, insbesondere N,N-Bis-(β-oxybutyl)-3,5-di-t-butylanilin sowie N,N-Bis-(hydroxyalkyl)-3,4,5-trimethylaniline.

Gut geeignete Aktivatoren sind auch die in der DE-B-14 95 520 beschriebenen Barbitursäuren und Barbitursäurederivate sowie die in der EP-A-0 059 451 beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Dioctyl-4-isobutylmalonylsulfamid.

Zur weiteren Beschleunigung wird die Polymerisation hierbei vorzugsweise in Gegenwart von Schwermetallverbindungen und ionogenem Halogen oder Pseudohalogen durchgeführt. Als Schwermetall ist Kupfer, als Halogenid das Chloridion besonders geeignet. Das Schwermetall wird geeigneterweise in Form löslicher organischer Verbindungen eingesetzt. Ebenso werden die Halogenidund Pseudohalogenidionen geeigneterweise in Form von löslichen Salzen eingesetzt, beispielsweise genannt seien die löslichen Aminhydrochloride sowie quartäre Ammoniumchloridverbindungen.

Wenn die erfindungsgemäßen Dentalmassen ein Redoxinitiatorsystem aus organischem Peroxid und Aktivator enthalten, so sind vorzugsweise Peroxid und Aktivator in räumlich voneinander getrennten Teilen der erfindungsgemäßen Dentalmasse vorhanden, die erst unmittelbar vor der Anwendung homogen miteinander vermischt werden. Liegen nebeneinander organisches Peroxid, Kupferverbindung, Halogenid und Malonylsulfamid und/oder Barbitursäure vor, so ist es insbesondere sinnvoll, dass organisches Peroxid, Malonylsulfamid und/oder Barbitursäure und die Kombination Kupferverbindung/Halogenid in drei räumlich voneinander getrennten Bestandteilen vorliegen. Beispielsweise können die Kombination Kupferverbindung/Halogenid, polymerisierbare Monomere sowie Füllstoffe zu einer Paste verknetet sein und die anderen Komponenten in oben beschriebener Weise jeweils mit einer geringen Menge an Füllstoffen oder insbesondere Thixotropie-Hilfsmitteln, wie silanisierter Kieselsäure, und einem Weichmacher, beispielsweise Phthalat, zu zwei separaten Pasten verknetet sein. Andererseits können die polymerisierbaren Monomeren auch zusammen mit organischem Peroxid und Füllern vorliegen. Alternativ ist auch eine Aufteilung von organischem Peroxid, Kupferverbindung, Halogenid und Malonylsulfamid und/oder Barbitursäure gemäß DE-A-199 28 238 realisierbar.

Als Vertreter der Komponente (K5) können beispielsweise zur Erhöhung der Flexibilität der Massen lösliche organische Polymere eingesetzt werden. Geeignet sind beispielsweise Polyvinylacetat sowie die Copolymeren auf Basis Vinylchlorid/Vinylacetat, Vinylchlorid/Vinylisobutylether und Vinylacetat/Maleinsäuredibutylether. Gut geeignet als zusätzliche Weichmacher sind beispielsweise Dibutyl-, Dioctyl- und Dinonylphthalate oder -adipate sowie höhermolekulare Polyphthalsäure- und Adipinsäureester. Als Thixotropiehilfsmittel können neben pyrogenen Kieselsäuren auch modifizierte Schichtsilikate (Bentonite) oder organische Modifikatoren, beispielsweise auf Basis hydrierter Rizinusöle eingesetzt werden. Als Additive können weiterhin Verzögerer, wie sie in der EP-A-0 374 824 als Komponente (d) beschrieben sind, in den Formulierungen enthalten sein.

Die erfindungsgemäßen Komponenten können beispielsweise auf zwei Pasten wie folgt verteilt sein:
- Paste 1: Teile von K3, Teile von K4, K5
- Paste 2: K1, K2, Teile von K3, Teile von K4.

Dentalmaterialien, die die erfindungsgemäßen Urethanpräpolymere enthalten, können beispielsweise als Füllungsmaterialien, Zemente, provisorische Kronenund Brückenmaterialien, Verblendkunststoffe, Prothesenmaterialien, kieferorthopädische Materialien, Kunststoffe zur Fissurenversiegelung, Modellierkunststoffe oder Modellkunststoffe verwendet werden.

Die erfindungsgemäßen Urethanpräpolymere und daraus hergestellte Formulierungen eignen sich auch für das Verkleben und Beschichten von Substraten. Exemplarisch sei die Beschichtung von Holz, Metall, Glas oder Plastik genannt. Weiterhin eignen sich die erfindungsgemäßen Urethanpräpolymere und daraus hergestellte Formulierungen beispielsweise auch zur Herstellung von Formkörpem.

Bevorzugt werden Massen, enthaltend die erfindungsgemäßen Urethanpräpolymere, als zweikomponentige Mischungen formuliert, wobei diese vorzugsweise in ein Kartuschensystem abgefüllt werden und die Massen über einen statischen oder dynamischen Mischaufsatz ausgebracht, homogen vermischt und ausgehärtet werden.

Im folgenden wird die Erfindung anhand von Beispielen näher beschrieben, wobei diese als die Erfindung in keinster Weise limitierend zu verstehen sind.

### Beispiele

### Herstellungsbeispiel 1: Urethanacrylat

In einem mit Thermometer, Rückflußkühler, mechanischem Rührer und Trockenrohr ausgestatteten 2I 3-Halskolben werden 212 g (ca. 0,2 Mol) Tego-Diol BD-1000, 70 g Essigsäureethylester und 0,1 g Dibutylzinndilaurat vorgelegt und unter Kühlung 126 g (0,6 Mol) Trimethyl-hexamethylendiisocyanat über einen Zeitraum von 30 Minuten zudosiert. Das Reaktionsgemisch wird 20 Stunden bei Raumtemperatur gerührt bis der Isocyanat-Gehalt auf 7,7% gefallen ist. Unter Kühlung werden 91 g Hydroxyethylacrylat zugegeben. Nach 72 Stunden bei Raumtemperatur wird ein Isocyanatwert von 0 ermittelt, womit die Reaktion beendet ist. Der Essigsäureethylester kann im Feinvakuum abdestilliert werden.

### Herstellungsbeispiel 2: Urethanmethacrylat

In einem mit Thermometer, Rückflußkühler, mechanischem Rührer und Trockenrohr ausgestatteten 2I 3-Halskolben werden 212 g (ca. 0,2 Mol) Tego-Diol BD-1000, 140 g Aceton und 0,1 g Dibutylzinndilaurat vorgelegt und unter Kühlung 84 g (0,4 Mol) Trimethyl-hexamethylendiisocyanat über einen Zeitraum von 30 Minuten zudosiert. Das Reaktionsgemisch wird 20 Stunden bei Raumtemperatur gerührt bis der Isocyanat-Gehalt auf 3,7% gefallen ist. Unter Kühlung werden 53 g Hydroxyethylmethacrylat zugegeben. Nach 72 Stunden bei Raumtemperatur wird ein Isocyanatwert von 0,1% ermittelt, womit die Reaktion beendet ist. Der Essigsäureethylester kann im Feinvakuum abdestilliert werden.

### Verwendungsbeispiele

Aus den in Tabelle 1 und Tabelle 2 aufgeführten Bestandteilen werden jeweils 10g Katalysator- und 100g Basispaste geknetet. Diese werden in 10 : 1-Kartuschen der Firma Mixpack, Rotkreuz gefüllt. Zur Anwendung werden sie mittels eines Dispensers durch eine statische Mischvorrichtung gedrückt und dabei vermischt, so dass die Aushärtung innerhalb von einigen Minuten eintritt.

**Tabelle 1:**

| **Katalysator** | | | |
|---|---|---|---|
| **Komponente** | **Bestandteil** | **Menge [g]** | **Gew.-%** |
| K3 | Fluoroaluminosilicatglaspulver (Ø<12µm) | 3,4 | 34 |
| K3 | Mikrofeine Kieselsäure silanisiert (HDKH 2000, Wacker, Burghausen) | 0,7 | 7 |
| K4 | 1-Benzyl-5-phenylbarbitursäure | 0,1 | 1 |
| K4 | 3,5,5-Trimethylhexansäure-tertiärbutylester | 0,06 | 0,6 |
| K5 | 2,2-Bis-4-(2-hydroxyethoxyphenyl)-propan-bis-acetat | 5,74 | 57,4 |

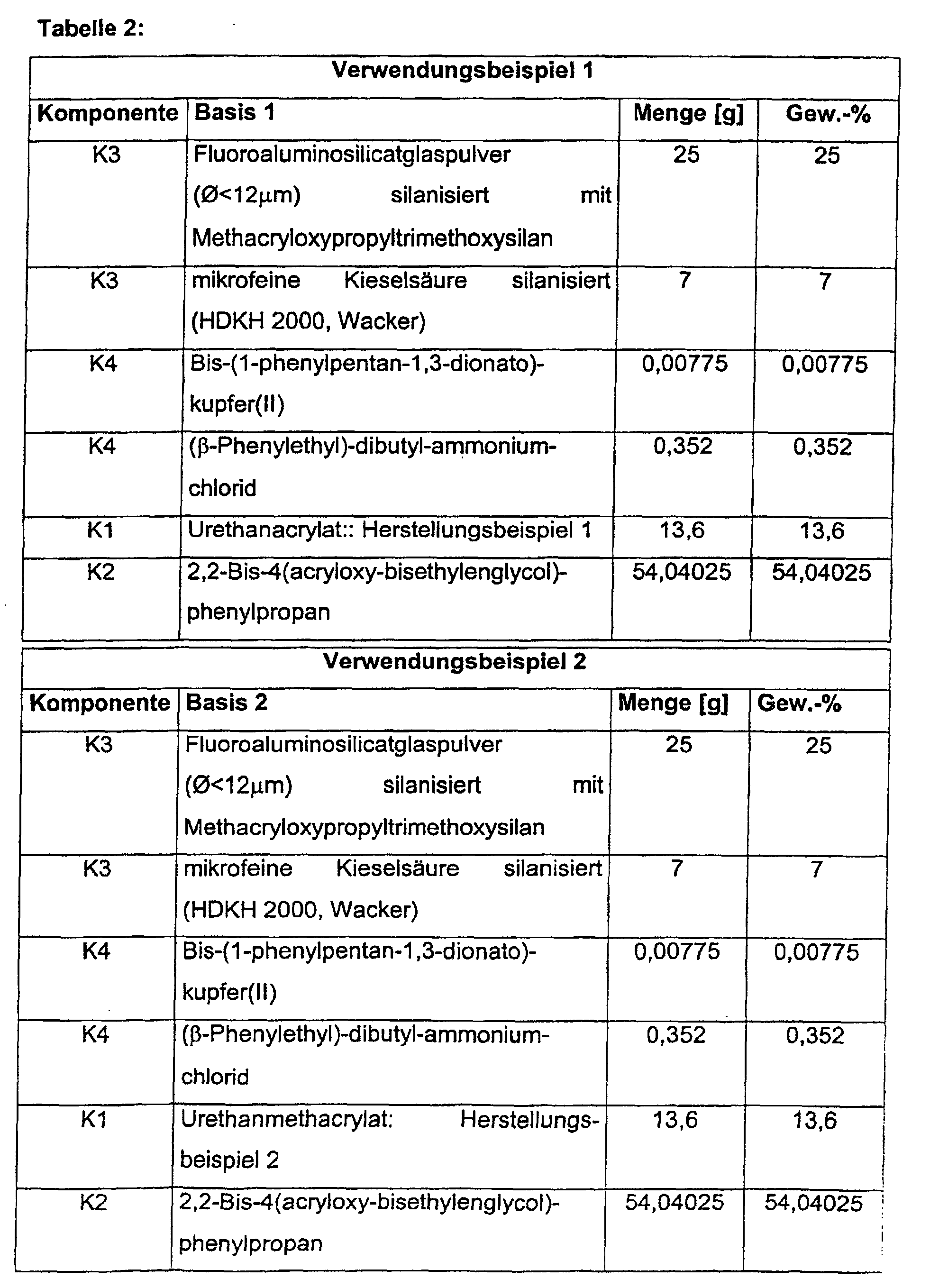

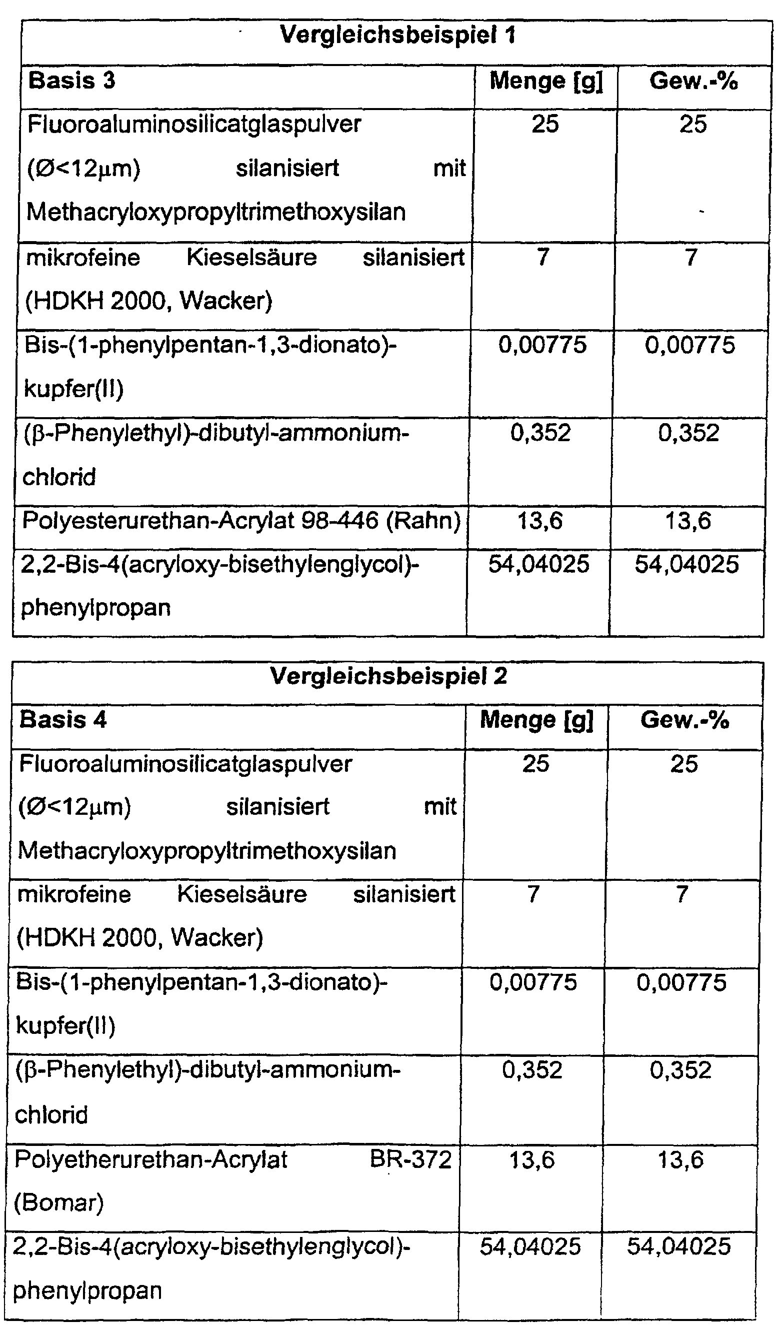

Mit den ausgehärteten Prüfkörpern wurden folgende Messwerte erhalten:

**Tabelle 3:**

| **Basis** | **Polyurethan- (meth)acrylat** | **Lineare Quellung, 7d in 36°C H**_{**2**}**O [%]** | **E-Modul [MPa]** | **Durchbiegung bei Bruch [mm]** | **Schlagzähigkeit [kJ/m**^{**2**}**]** |
|---|---|---|---|---|---|
| 1 | Urethanacrylat: Herstellungsbeispiel 1 | 0,2 | 1450 | 1,7 | 9,41 |
| 2 | Urethanmethacrylat Herstellungsbeispiel 2 | 0,25 | 1200 | 1.3 | 9,97 |
| 3 | Polyesterurethan-Acrylat 98-446 (Rahn) | 0,35 | 900 | 1,4 | 4,89 |
| 4 | Polyetherurethan-Acrylat BR-372 (Bomar) | 0,4 | 1000 | 1,2 | 4,42 |

- Meßmethoden:: E-Modul und Durchbiegung: 3-Punktbiegeversuch;
Schlagzähigkeit = Kerbschlagzähigkeit;
lineare Quellung nach Lagerung von Prüfkörpern für 7 Tage in Wasser bei 36°C; Angabe in % der Ausgangslänge.

Die mit den nicht-erfindungsgemäßen Basispasten 3 und 4 hergestellten Prüfkörper zeigen im Vergleich zu den Prüfkörpern aus erfindungsgemäßen Pasten (Basis 1 und 2) eine erhöhte Quellung, ein erniedrigtes E-Modul und eine stark erniedrigte Schlagzähigkeit.

## Patentansprüche

1. Urethanpräpolymere, erhältlich durch Umsetzung von:
(A) 15 bis 85 Gew.-% eines oder mehrerer α,ω-terminierter Poly(meth)acrylatdiole,
(B) 0 bis 30 Gew.-% einer oder mehrerer radikalisch härtbarer, polyhydroxyfunktioneller Verbindungen,
(C) 14 bis 60 Gew.-% eines oder mehrerer Polyisocyanate,
(D) 1 bis 40 Gew.-% einer gegenüber Isocyanatgruppen reaktiven monofunktionellen Verbindung, die zusätzlich über eine oder mehrere radikalisch härtbare Gruppierungen verfügt.

2. Urethanpräpolymere gemäß Anspruch 1, wobei die Vertreter der Komponente (B) OH-Zahlen von 40 bis 700 mg KOH/g aufweisen, insbesondere polyhydroxygruppenhaltige Polyester(meth)acrylatpräpolymere, polyhydroxygruppenhaltige Polyepoxy(meth)acrylatpräpolymere, polyhydroxygruppenhaltige Polyurethan(meth)acrylatpräpolymere.

3. Urethanpräpolymere gemäß einem der Ansprüche 1 oder 2, wobei die Vertreter der Komponente (C) aliphatischer, cycloaliphatischer und/oder aromatischer Natur sind und mindestens zwei freie Isocyanatgruppen aufweisen.

4. Urethanpräpolymere gemäß einem der Ansprüche 1 bis 3, wobei die Vertreter der Komponente (C) Diisocyanate X(NCO)₂ sind, wobei X einen aliphatischen Kohlenwasserstoffrest mit 2 bis 12 C-Atomen, einen cycloaliphatischen Kohlenwasserstoffrest mit 5 bis 18 C-Atomen, einen aromatischen Kohlenwasserstoffrest mit 6 bis 16 C-Atomen und/oder einen araliphatischen Kohlenwasserstoffrest mit 7 bis 15 C-Atomen bedeutet.

5. Urethanpräpolymere gemäß einem der Ansprüche 1 bis 4, wobei die Vertreter der Komponente (D) eine Hydroxygruppe aufweisen und als radikalisch härtbare Gruppierungen (Meth)acrylat-Gruppen besitzen.

6. Verwendung der Urethanpräpolymere gemäß einem der Ansprüche 1 bis 5 zur Herstellung von Dentalmassen sowie zum Verkleben, Beschichten oder Vergießen von Substraten, insbesondere zur Herstellung von Füllungsmaterialien, Stumpfaufbaumaterialien, Befestigungszementen, provisorischen Kronen- und Brückenmaterialien, zahntechnischen Werkstoffen, Modellmaterialien, Inlays, Onlays, Verblendschalen, Kronen und Brücken.

7. Zusammensetzungen, enthaltend:
(K1) 1 bis 70 Gew.-% mindestens eines Urethanpräpolymers gemäß einem der Ansprüche 1 bis 5,
(K2) 8,9 bis 70 Gew.-% eines oder mehrerer radikalisch polymerisierbarer Monomerer,
(K3) 10 bis 90 Gew.-% Füllstoffe,
(K4) 0,1 bis 5 Gew.-% Initiatoren und gegebenenfalls Aktivatoren,
(K5) 0 bis 30 Gew.-% Additive, gegebenenfalls Pigmente Thixotropiehilfsmittel, Weichmacher.

8. Zusammensetzungen gemäß Anspruch 7, wobei als Komponente (K2) mono-, di- und/oder höherfunktioneller Acrylsäure- und/oder Methacrylsäureester eingesetzt werden, die sowohl monomere als auch höhermolekulare oligomere oder polymere Acrylate beinhalten können und in alleiniger Form oder in Abmischung eingesetzt werden.

9. Verwendung der Zusammensetzungen gemäß einem der Ansprüche 7 bis 8 im Dentalbereich, insbesondere als Füllungsmaterial. Stumpfaufbaumaterial, Befestigungszement, provisorisches Kronen- und Brückenmaterial, zahntechnischer Werkstoff, Modellmaterial, Inlay, Onlay, Verblendschale, Kronen und Brücken.

10. Kit zur Herstellung eines Dentalmaterials, enthaltend gegebenenfalls ein Applikationsgerät zur Ausbringung von Dentalmaterialien, gegebenenfalls einen statischen Mischaufsatz, mindestens eine Kartusche mit mindestens zwei Kammern, die mit einer Zusammensetzung nach einem der Ansprüche 7 bis 8 gefüllt sind.

11. Ausgehärtete Zusammensetzung nach einem der Ansprüche 7 bis 8.

12. Applikationsgerät, enthaltend mindestens eine Kartusche, enthaltend eine Zusammensetzung nach einem der Ansprüche 7 bis 8.

## Claims

1. Urethane prepolymers, obtainable by reaction of:
(A) 15 to 85 wt.-% of one or more α,ω-terminated poly(meth)acrylate diols,
(B) 0 to 30 wt.-% of one or more radically curable, polyhydroxy-functional compounds,
(C) 14 to 60 wt.-% of one or more polyisocyanates,
(D) 1 to 40 wt.-% of a monofunctional compound, reactive vis-à-vis isocyanate groups, which also contains one or more radically curable groupings.

2. Urethane prepolymers according to claim 1, the representatives of the component (B) displaying OH numbers from 40 to 700 mg KOH/g, in particular polyhydroxy-group-containing polyester(meth)acrylate prepolymers, polyhydroxy-group-containing polyepoxy(meth)acrylate prepolymers, polyhydroxy-group-containing polyurethane(meth)acrylate prepolymers.

3. Urethane prepolymers according to one of claims 1 or 2, the representatives of the component (C) being of an aliphatic, cycloaliphatic and/or aromatic nature and displaying at least two free isocyanate groups.

4. Urethane prepolymers according to one of claims 1 to 3, the representatives of the component (C) being diisocyanates X(NCO)₂, X representing an aliphatic hydrocarbon radical with 2 to 12 C atoms, a cycloaliphatic hydrocarbon radical with 5 to 18 C atoms, an aromatic hydrocarbon radical with 6 to 16 C atoms and/or an araliphatic hydrocarbon radical with 7 to 15 C atoms.

5. Urethane prepolymers according to one of claims 1 to 4, the representatives of the component (D) having a hydroxy group and, as radically curable groupings, (meth)acrylate groups.

6. Use of the urethane prepolymers according to one of claims 1 to 5 for the preparation of dental compounds as well as for the gluing, coating or pouring of substrates, in particular for the preparation of filling materials, stump build-up materials, fixing cements, temporary crown and bridge materials, dental materials, model materials, inlays, onlays, facing shells, crowns and bridges.

7. Compositions, containing:
(C1) 1 to 70 wt.-% of at least one urethane prepolymer according to one of claims 1 to 5,
(C2) 8.9 to 70 wt.-% of one or more radically polymerizable monomers,
(C3) 10 to 90 wt.-% fillers,
(C4) 0.1 to 5 wt.-% initiators and optionally activators,
(C5) 0 to 30 wt.-% additives, optionally pigments, thixotropic auxiliaries, plasticizers.

8. Compositions according to claim 7, mono-, di- and/or higher-functional acrylic acid and/or methacrylic acid esters being used as component (C2), which can contain both monomeric and higher-molecular oligomeric or polymeric acrylates and are used alone or in a mixture.

9. Use of the compositions according to one of claims 7 to 8 in the dental field, in particular as filling material, stump build-up material, fixing cement, temporary crown and bridge material, dental material, model material, inlay, onlay, facing shell, crowns and bridges.

10. Kit for the preparation of dental materials optionally containing an applicator for the dispensing of dental materials, optionally a static mixing set, at least one cartouche with at least two chambers which are filled with a composition according to one of claims 7 to 8.

11. Cured composition according to one of claims 7 to 8.

12. Applicator, containing at least one cartouche, containing a composition according to one of claims 7 to 8.

## Revendications

1. Prépolymère d'uréthane, obtenu par transformation de
(A) de 15 à 85 % en poids d'un ou de plusieurs diols α, ω-terminaux de poly(méth)acrylate ;
(B) 0 à 30 % en poids d'un ou de plusieurs composés polyhydroxy fonctionnels à durcissement radicalaire ;
(C) 14 à 60 % en poids d'un ou de plusieurs polyisocyanate ;
(D) 1 à 40 % en poids d'un composé monofonctionnel réagissant par rapport aux groupes d'isocyanate, lequel composé dispose en outre d'un ou de plusieurs regroupements à durcissement radicalaires.

2. Prépolymère d'uréthane selon la revendication 1, dans lequel le représentant du composant (B) présente une quantité de OH de 40 à 700 mg KOH/g, et en particulier des prépolymères de polyester(méth)acrylate contenant des groupes polyhydroxy, des prépolymères de polyépoxy(méth)acrylate contenant des groupes polyhydroxy.

3. Prépolymère d'uréthane selon l'une quelconque des revendications 1 ou 2, dans lequel les représentants du composant (C) sont de nature aliphatique, cycloaliphatique et / ou aromatique et présente au moins deux groupes d'isocyanate libres.

4. Prépolymère d'uréthane selon l'une quelconque des revendications 1 ou 2, dans lequel les représentants du composant (C) sont du diisocyanate x(NCO)₂, où X désigne un résidu d'hydrocarbure aliphatique avec 2 à 12 atomes de carbone, un résidu d'hydrocarbure cycloaliphatique avec 5 à 18 atomes de carbone, un résidu d'hydrocarbure aromatique avec 6 à 16 atomes de carbone et / ou un résidu d'hydrocarbure araliphatique avec 7 à 15 atomes de carbones.

5. Prépolymère d'uréthane selon l'une des revendications 1 à 4, dans lequel les représentants des composants (D) présentent un groupe hydroxy et possèdent des groupes de (méth)acrylate comme groupements à durcissement radicalaire.

6. Utilisation des prépolymères d'uréthane selon l'une quelconque des revendications 1 à 5 permettant la fabrication des masses dentaires ainsi que le collage, le revêtement ou le scellement des substrats, et notamment la fabrication de matériau de remplissage, des matériaux pour faux-moignon, des ciments de fixation, des matériaux de bridges et couronnes provisoires, des matériaux pour les techniques dentaires des matériaux pour les modèles, inlays, onlays, les coiffes de protection, les couronnes et les bridges.

7. Compositions, contenant :
(K1) 1 à 70 % en poids au moins d'un prépolymère d'uréthane selon l'une quelconque des revendications 1 à 5 ;
(K2) 8,9 à 70 % en poids d'un ou plusieurs monomères polymérisables au niveau du radical,
(K3)10 à 90 % en poids de matériau de remplissage,
(K4) 0,1 à 5 % en poids d'initiateurs et le cas échéant d'activateurs
(K5) 0 à 30 % en poids d'additifs, le cas échéant de pigments, de remèdes de thixotropie, de fluidisants.

8. Compositions selon la revendication 7, dans lesquelles des esters d'acides méthacryliques, et / ou d'acides acryliques extrêmement fonctionnels et / ou des diesters, des monoesters, sont utilisés comme composants (K2), lesquels esters peuvent contenir aussi bien des monomères que des oligomères extrêmement moléculaires ou des acrylates polymères et lesquels peuvent être utilisés sous toutes les formes et dans tout mélange.

9. Utilisation des compositions selon l'une quelconque des revendications 7 à 8 dans le secteur dentaire, notamment matériau de remplissage, matériau pour faux-moignon, ciment de fixation, matériau de bridges et couronnes provisoires, matériau pour les techniques dentaires, matériau pour les modèles, inlay, onlay, coiffes de protection, couronnes et bridges.

10. Kit de fabrication d'un matériau dentaire, contenant le cas échéant un appareil d'application pour produire des matériaux dentaires, le cas échéant, un dispositif de mélange statique, au moins une cartouche avec au moins deux chambres, qui sont remplies d'une composition selon l'une des revendications 7 à 8.

11. Composition durcie selon l'une des revendications 7 à 8.

12. Appareil d'application, contenant au moins une cartouche contenant une composition selon l'une des revendications 7 à 8.
